# EUROPEAN PATENT SPECIFICATION

(11) **EP 2 394 620 B1**
(45) Date of publication and mention of the grant of the patent: **12.09.2018**
(21) Application number: 10738442.2
(22) Date of filing: 27.01.2010
(51) Int. Cl.: A61F 13/15, A61F 13/472, A61F 13/49

(54) **APPARATUS FOR MANUFACTURING AN ABSORBING BODY AND METHOD OF MANUFACTURING AN AIR PERMEABLE MEMBER**
VORRICHTUNG ZUR HERSTELLUNG EINES AUFNAHMEFÄHIGEN KÖRPERS UND VERFAHREN ZUR HERSTELLUNG EINES LUFTDURCHLÄSSIGEN ELEMENTS
DISPOSITIF DE FABRICATION DE CORPS ABSORBANT ET PROCÉDÉ DE FABRICATION DUN ÉLÉMENT PERMÉABLE À L'AIR

(30) Priority: 05.02.2009 JP 2009025228
(43) Date of publication of application: 14.12.2011
(73) Proprietor: Unicharm Corporation, Ehime 799-0111 (JP)
(72) Inventor: ISHIKAWA, Masahiko, Kanonji-shi Kagawa 769-1602 (JP)
(74) Representative: Staeger & Sperling Partnerschaftsgesellschaft mbB
(86) International application number: PCT/JP2010/051021
(87) International publication number: WO 2010/090107

(56) References cited:
- WO-A1-2007/037357
- JP-A- 2 107 250
- JP-A- 7 119 013
- JP-A- 2006 122 109
- JP-A- 2009 000 386
- US-A- 4 242 176
- US-A- 4 666 647
- US-A- 4 761 258
- US-A- 4 936 934
- US-A- 5 398 816

## Description

### Technical Field

The invention relates to a manufacturing apparatus for an absorbent body associated with absorbent article such as a disposable diaper, and a method for manufacturing an air-permeable member that is disposed of the manufacturing apparatus to form an absorbent body.

### Background Art

Disposable diapers, sanitary napkins or the like are used, as an example of an absorbent article that absorbs exudates such as urine or menstrual blood. These absorbent articles include an absorbent body provided by forming pulp fibers, an example of a liquid absorbent material, in predetermined shape.

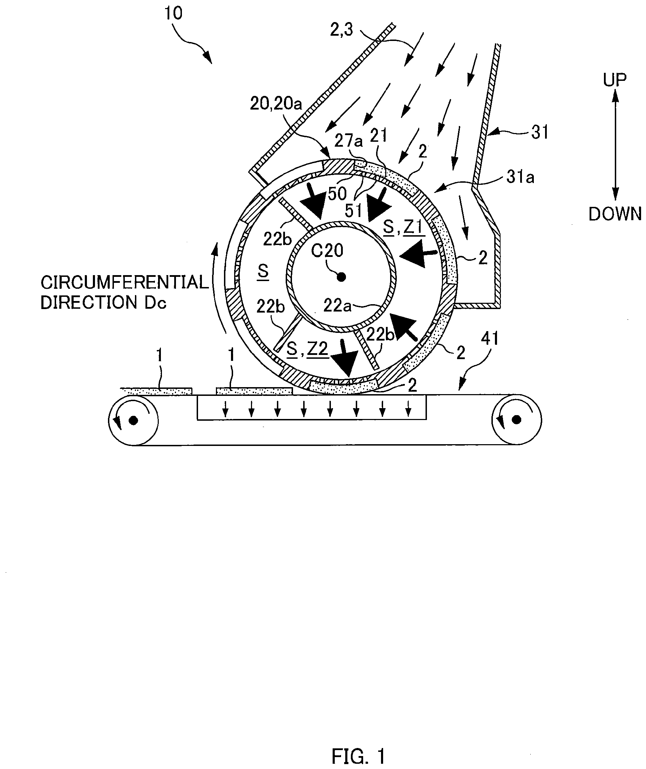

An absorbent body 1 is formed by a fiber depositing apparatus 10 in a manufacturing line (See FIG. 1). The fiber depositing apparatus 10 includes a rotatable drum 20. With rotating the rotatable drum 20 in a circumferential direction Dc, mixed air 3 in which pulp fibers 2 are entrained is supplied towards an outer circumferential face 20a of the drum and pulp fibers 2 are deposited on a mold 21 that is of a depression in the outer circumferential face 20a. Thereafter, the deposited pulp fibers 2 are removed from the mold 21, and the absorbent body 1 is formed.

The mold 21 is formed by affixing a woven wire cloth or the like serving as an air-permeable member 50 on an opening 27a formed on the outer circumferential face 20a of the rotatable drum 20, inside the rotatable drum 20, for example. While depositing, the mixed air 3 is sucked through the woven wire cloth 50 from the outside of the rotatable drum 20 to the inside of the drum 20, and the pulp fibers 2 are deposited on the woven wire cloth 50. While removing, air is discharged through the woven wire cloth 50 from the inside of the rotatable drum 20 to the outside of the drum 20; therefore, the pulp fibers 2 deposited in the mold 21 is removed as the absorbent body 1.

Therefore, the woven wire cloth 50 undergoes an external force that acts in the directions opposite to each other while depositing and removing. In other words, every time a single absorbent body 1 is formed, the woven wire cloth 50 is forced to undergo deformation in which the bending direction is repeatedly inverted.

Herein, the woven wire cloth 50 is generally made of thin metal wire, and stiffness thereof is low. If external forces whose directions are opposite to each other act on the woven wire cloth 50 repeatedly, there is a possibility that fatigue failure occurs to woven wire cloth 50 easily. Therefore, woven wire cloth 50 is disclosed that a reinforcing member is joined thereto in order to suppress deformation of the woven wire cloth 50 (See PTL 1, for example).

### Citation List

### [Patent Literature]

[PTL 1] Japanese Patent Application Laid-open Publication No.62-206071 corresponding to US 4,666,647.

Further, US 4,761,258 discloses an apparatus for forming a fibrous web having a web forming layer, a foraminous spacing means and a gas flow regulating layer.

### Summary of the Invention

### Technical Problem

However, PTL 1 does not disclose at which portion of the reinforcing member the reinforcing member and the woven wire cloth 50 are joined. Further, if the reinforcing member is joined to the woven wire cloth 50 at a comparatively low rigidity portion of the reinforcing member, there is a possibility that desirable reinforcement effect is not achieved because the aforementioned low rigidity portion is deformed easily by the aforementioned external force which acts on the woven wire cloth 50 while depositing and removing so that fatigue failure occurs to the reinforcing member itself. In other words, there is a possibility that deformation of the woven wire cloth 50 is not effectively suppressed.

This invention has been made in view of the above problems, and an advantage thereof is to provide a reinforcing member that reinforces securely an air-permeable member such as a woven wire cloth or the like and suppresses deformation of the air-permeable member.

### Solution to Problem

An aspect of the invention to achieve the above advantage is
a manufacturing apparatus according to claim 1.

Further,
a method for manufacturing an air permeable member that is used when forming an absorbent body associated with an absorbent article by depositing a liquid absorbent material, that is reinforced by a reinforcing member, and that provides at least either one of a depression and a protrusion having a shape corresponding to a target shape of the absorbent body, wherein
a material of the air-permeable member is a member regulating passage of the liquid absorbent material in a thickness direction,
a material of the reinforcing member is a mesh in which a first wire and a second wire that intersect are connected at an intersection point of these wires, and
the method including:
joining and integrating the material of the air-permeable member and the material of the reinforcing member by diffusion welding the material of the reinforcing member to the material of the air-permeable member under inert gas atmosphere at the intersection point of the first wire and the second wire while superimposing in the thickness direction the material of the air-permeable member and the material of the reinforcing member; and
forming at least either one of the depression and the protrusion by pressing the joined and integrated materials of the reinforcing member and the air-permeable member while sandwiching the materials between a male press mold and a female press mold.

Other features of this invention will become apparent from the description in this specification and the attached drawings.

### Effects of the Invention

According to this invention, it is possible that a reinforcing member reinforces securely an air-permeable member and deformation of the air-permeable member is suppressed.

### BRIEF DESCRIPTION OF THE DRAWINGS

FIG. 1 is a sectional view showing an example of the manufacturing apparatus 10 of the absorbent body 1, the equipment 10 herein being cut on its center along the longitudinal direction thereof.
FIG. 2 is an exploded perspective view of a rotatable drum 20.
FIG. 3 is an exploded perspective view of a mold plate 27.
FIG. 4A is an enlarged plan view showing an air-permeable member 50 that is partially omitted herein, and FIGS. 4B and 4C are respectively a B-B cross-sectional view and a C-C cross-sectional view in FIG. 4A.
FIG. 5A is an explanatory diagram of the first embodiment; a plan view showing the air-permeable member 50 that is partially omitted herein, and FIGS. 5B and 5C are respectively a B-B cross-sectional view and C-C cross-sectional view in FIG. 5A.
FIG. 6A is a plan view showing positions at which a depression 56a and a protrusion 56b are formed in the air-permeable member 50 and a reinforcing member 60, and FIG. 6B is a longitudinal sectional view taken along B-B line in FIG. 6A.
FIGS. 7A and 7B are explanatory diagrams of the modified example of the first embodiment, longitudinal sectional views corresponding respectively to a B-B cross section and a C-C cross section in FIG. 5A.
FIG. 8A is an explanatory diagram of the second embodiment, a plan view showing an air-permeable member 50a that is partially omitted herein, and FIGS. 8B and 8C are respectively a B-B cross-sectional view and a C-C cross-sectional view in FIG. 8A.
FIG. 9A is a plan view showing positions at which a low density region 57a and a depression 57b of an air-through hole 51a is formed in the air-permeable member 50a, and FIG. 9B is a longitudinal sectional view taken along B-B line in FIG. 9A.
FIG. 10A is a plan view of an air-permeable member 50b or a reinforcing member 60b that are woven in twill weave, an example of other embodiments, and FIGS. 10B and 10C are respectively a B-B cross-sectional view and a C-C cross-sectional view in FIG. 10A.
FIG. 11 is a plan view of a reinforcing member 60b in which a first wire 61 and a second wire 62 are not woven, an example of the other embodiments.

### Mode for Carrying Out the Invention

With the description of this specification and the attached diagrams, at least the following will become clear.

A manufacturing apparatus for an absorbent body associated with an absorbent article, the manufacturing apparatus manufacturing the absorbent body using a procedure in which a liquid absorbent material is deposited on an air-permeable member by passing air that contains the liquid absorbent material in a thickness direction of the air-permeable member, the air-permeable member covering an opening of a mold member, including: a reinforcing member for reinforcing the air-permeable member by being superimposed on the air-permeable member in the thickness direction, the reinforcing member being a mesh formed by connecting a first wire and a second wire that intersect, at an intersection point of these wires, and the reinforcing member being joined to the air-permeable member at the intersection point.

According to such a manufacturing apparatus for an absorbent body, the reinforcing member is joined to the air-permeable member at the intersection point of the first wire and the second wire. The intersection point is a portion having relatively higher rigidity than a portion formed by the first wire or second wire alone because the first wire and second wire are connected at the intersection point; in other words, the intersection point is a portion that is less likely to deform. As a result, the intersection point can sufficiently resist an external force that acts on an air-permeable member while depositing the absorbent body, so that deformation of the air-permeable member is surely suppressed.

In addition, since the air-permeable member and the reinforcing member are joined and integrated at the intersection point, these members deform substantially integrally. Therefore, a distance between the air-permeable member and the reinforcing member is more likely to be held constant. As a result, even if the liquid absorbent material is sandwiched between the air-permeable member and the reinforcing member, the liquid absorbent material is likely to be released and clogging of a mesh opening of the air-permeable member or the reinforcing member can be prevented effectively.

In this manufacturing apparatus for an absorbent body, it is desirable that the first wire and the second wire be connected at the intersection point by weaving.
According to such a manufacturing apparatus for an absorbent body, the first wire and the second wire can be connected firmly.

In this manufacturing apparatus for an absorbent body, it is desirable that the first wire and the second wire be joined at the intersection point.

According to such a manufacturing apparatus for an absorbent body, the first wire and the second wire are joined. This enables rigidity of the reinforcing member itself to increase, and deformation thereof is suppressed. As a result, fatigue failure becomes less likely to occur. In addition, because deformation of the mesh opening of the reinforcing member is also suppressed, the liquid absorbent material is also prevented effectively from being caught by and clogging the mesh opening due to deformation of the opening.

In this manufacturing apparatus for an absorbent body, it is desirable that the air-permeable member be a mesh formed by weaving a third wire and a fourth wire that intersect, a mesh opening of the air-permeable member be finer than a mesh opening of the reinforcing member, an intersection point of the third wire and the fourth wire be arranged corresponding to all of an intersection point that is of the reinforcing member and is positioned in the opening, and the intersection point of the reinforcing member and the corresponding intersection point of the third wire and the fourth wire be matched up.

According to such a manufacturing apparatus for an absorbent body, the intersection points of the third wire and the fourth wire is arranged corresponding to all of the intersection points that the reinforcing member has in the opening of the mold member. Further, the intersection points corresponding to each other are matched up. Therefore, the high-rigidity joint at which the intersection points are matched up makes it possible to effectively suppress deformation of the air-permeable member more surely across wider area.

In this manufacturing apparatus for an absorbent body, it is desirable that the air-permeable member be a mesh formed by weaving a third wire and a fourth wire that intersect, the air-permeable member and the reinforcing member have at least either one of a depression and a protrusion that are formed by pressing while being superimposed on and joined to each other in the thickness direction, and prior to the pressing, the first wire be arranged at a first pitch in a longitudinal direction of the second wire, the second wire be arranged at a second pitch in a longitudinal direction of the first wire, a longitudinal direction of the third wire and the longitudinal direction of the first wire be aligned, a longitudinal direction of the fourth wire and the longitudinal direction of the second wire be aligned, the third wire be arranged at a third pitch in the longitudinal direction of the fourth wire, the fourth wire be arranged at a fourth pitch in the longitudinal direction of the third wire, the first pitch be integer multiple (equal to or more than 2 times) of the third pitch, and the second pitch be integer multiple (equal to or more than 2 times) of the fourth pitch.

According to such a manufacturing apparatus for an absorbent body, the intersection points of the air-permeable member can be surely matched with all intersection points that the reinforcing member has in the opening and be arranged. As a result, deformation of the air-permeable member is effectively suppressed across wide range.

In addition, the air-permeable member includes the depression and the protrusion that are formed by pressing. This enables an absorbent body to be formed in a three dimensional shape having a localized region with a different weight of fibers per unit area in the absorbent body (g/cm²).

In this manufacturing apparatus for an absorbent body, it is desirable that the third wire and the fourth wire be joined at an intersection point of the third wire and the fourth wire.

According to such a manufacturing apparatus for an absorbent body, the third wire and the fourth wire are joined. This enables rigidity of the air-permeable member itself to increase, and deformation thereof is suppressed. As a result, fatigue failure is less likely to occur. In addition, because deformation of mesh is also suppressed, the liquid absorbent material is also prevented effectively from being caught by and clogging the mesh opening due to deformation of the opening.

In this manufacturing apparatus for an absorbent body, it is according to the invention that a main body of the air-permeable member is a plate member, and an air-through hole be formed along the thickness direction completely through the plate member.

According to such a manufacturing apparatus for an absorbent body, the air-permeable member is a plate member; therefore, comparing with the case where the air-permeable member is a woven cloth, portions surrounding the air-through holes are flat. As a result, clogging of the air-through holes of the liquid absorbent material is reduced. If, particularly, the liquid absorbent material is a grain such as superabsorbent polymer or the like, clogging of the air-through holes is effectively prevented.

In addition, compared with the case where the air-permeable member is a woven cloth, the degree of freedom is better with respect to design of the shape or position of the air-through holes.

In this manufacturing apparatus for an absorbent body, it is desirable that the plate member have a low density region in which a density of the air-through hole is lower than a surrounding region, within the opening.

According to such a manufacturing apparatus for an absorbent body, the plate member includes the low density region in which density of the air-through holes is low. In the low density region, the large number of points at which the plate member is joined to the reinforcing member can be ensured, compared with other regions surrounding the low density region. Therefore, compared with the case where the density of the air-through holes is uniform, joining stiffness of the reinforcing member can increase throughout the opening.

In addition, the weight of the liquid absorbent material per body having a targeted three dimensional shape.

In this manufacturing apparatus for an absorbent body, it is desirable that the joining be performed by diffusion welding under inert gas atmosphere.

According to such a manufacturing apparatus for an absorbent body, joining can be performed without brazing filler metal such as solder, filler rod or the like. This makes it possible to prevent lump of a brazing filler metal from being sandwiched with the mesh or the like, and to maintain good air-permeability of the air-permeable member, the reinforcing member or the like.

In addition, diffusion welding is performed under inert gas atmosphere. Therefore, when forming the depression or the protrusion by pressing on the air-permeable member and reinforcing member that are joined and integrated, rapid plastic deformation of the air-permeable member and reinforcing member can be achieved with maintaining the joined and integrated state thereof. Therefore, it is possible to avoid troubles, for example, the joint of the reinforcing member and air-permeable member breaks at the intersection points of the reinforcing member while pressing.

In this manufacturing apparatus for an absorbent body, it is desirable that the manufacturing apparatus further include a cylindrical rotatable drum that rotates continuously in a circumferential direction, the mold member form an outer circumferential face of the rotatable drum, an inner space and an outer space of the rotatable drum be in air-permeably communication through the air-permeable member, a supply duct that supplies the air towards the outer circumferential face of the rotatable drum from outside be provided at a first position of the circumferential direction, a pressure of an inner space that is of the rotatable drum and corresponds to the first position be maintained at negative pressure that is lower than that of the outer space, when the mold member passes the first position, the air in the supply duct is sucked through the air-permeable member into the inner space of the rotatable drum and the absorbent body be deposited on the air-permeable member, a removal position at which the absorbent body is removed from the air-permeable member be set at a position downstream from the first position in the circumferential direction, and a pressure of an inner space that is of the rotatable drum and corresponds to the removal position be maintained at a pressure equal to or higher than that of the outer space.

According to such a manufacturing apparatus for an absorbent body, it is possible to effectively achieve the foregoing operation and effect. In other words, when the mold member passes the first position, the air-permeable member is drawn by air, the drawing force acts radially inwardly in the rotation of the rotatable drum. On the contrary, when the mold member passes the removal position, the air-permeable member is drawn by air, and the drawing force acts radially outwardly in the rotation of the rotatable drum. In this way, the air-permeable member undergoes repeatedly an external force whose direction is inverted, and therefore there is a possibility that fatigue failure finally occurs to the air-permeable member. In terms thereof, the air-permeable member is joined to and integrated with the reinforcing member, and, in addition, the air-permeable member is joined at the intersection point that is particularly high-rigidity portion in the reinforcing member. Therefore, deformation of the air-permeable member when the member undergoes the aforementioned external force is effectively suppressed so that fatigue failure is less likely to occur.

Further,
A method for manufacturing an air-permeable member that is used when forming an absorbent body associated with an absorbent article by depositing a liquid absorbent material, that is reinforced by a reinforcing member, and that provides at least either one of a depression and a protrusion having a shape corresponding to a target shape of the absorbent body, wherein a material of the air-permeable member is a member regulating passage of the liquid absorbent material in a thickness direction, a material of the reinforcing member is a mesh in which a first wire and a second wire that intersect are connected at an intersection point of these wires, and the method including: joining and integrating the material of the air-permeable member and the material of the reinforcing member by diffusion welding the material of the reinforcing member to the material of the air-permeable member under inert gas atmosphere at the intersection point of the first wire and the second wire while superimposing in the thickness direction the material of the air-permeable member and the material of the reinforcing member; and forming at least either one of the depression and the protrusion by pressing the joined and integrated materials of the reinforcing member and the air-permeable member while sandwiching the materials between a male press mold and a female press mold.

According to such a method for manufacturing an air-permeable member, after the air-permeable member and the reinforcing member are joined and integrated with being superimposed, the depression or the protrusion or the like are formed by pressing. Compare with the case where the processes are performed in an inverted sequence, in other words, the case where, after the depression or the protrusion is formed on the members respectively, the air-permeable member and reinforcing member are joined with being superimposed. This makes it possible to prevent polymerization defects caused by poor forming accuracy or the like of the depression or the protrusion. As a result, joint failure of the air-permeable member and the reinforcing member can be prevented surely.

In addition, diffusion welding between the air-permeable member and the reinforcing member is performed under inert gas atmosphere. Therefore, when pressing the depression or the protrusion onto the air-permeable member and reinforcing member that are joined and integrated, rapid plastic deformation of the air-permeable member and reinforcing member can be achieved with maintaining the joined and integrated state thereof. As a result, it is possible to avoid troubles, for example, breaking the joining to the air-permeable member at the intersection points of the reinforcing member when pressing.

In a method for manufacturing an air-permeable member, it is desirable that the reinforcing member be a mesh formed by weaving the first wire and the second wire, the air-permeable member be a mesh formed by weaving a third wire and a fourth wire that intersect, in joining and integrating, the first wire and the second wire be joined by diffusion welding at the intersection point of the first wire and the second wire, and the third wire and the fourth wire be joined by diffusion welding at an intersection point of the third wire and the fourth wire.

According to such a method for manufacturing an air-permeable member, the reinforcing member is a woven cloth by weaving the first wire and second wire. In joining and integrating, the first wire and second wire that form this woven cloth are joined. This enables rigidity of the reinforcing member to increase, and deformation thereof is suppressed. As a result, fatigue failure is less likely to occur. In addition, because deformation of mesh of the reinforcing member is also suppressed, the liquid absorbent material is also prevented effectively from being caught by and clogging the mesh opening due to deformation of the opening.

In addition, the air-permeable member is a woven cloth, and in the joining-and-integrating step, the third wire and fourth wire that forms the woven cloth are joined. This enables to increase rigidity of the air-permeable member itself, deformation thereof is suppressed, and fatigue failure is less likely to occur. In addition, because deformation of mesh is also suppressed, the liquid absorbent material is also prevented effectively from being caught by and clogging the mesh opening due to deformation of the opening.

Further, employing diffusion welding makes it possible to join without brazing filler metal. Therefore, lump of a brazing filler metal is not sandwiched with the mesh or the like, and it is possible to maintain good air-permeability of the air-permeable member or the reinforcing member.

### === Present Embodiment ===

### <<< Overall Configuration of Manufacturing apparatus 10 for absorbent body 1 >>>

FIG. 1 is a sectional view showing an example of a manufacturing apparatus 10 for an absorbent body 1, the equipment 10 herein being cut on its center along the longitudinal direction thereof.

The manufacturing apparatus 10 of the absorbent body 1 is a so-called fiber depositing apparatus in which the absorbent body 1 is formed by depositing the pulp fibers 2 as a liquid absorbent material. The main configuration of the manufacturing apparatus 10 includes, for example, (1) a rotatable drum 20 that continuously rotates about a horizontal axis C20 in one direction, a circumferential direction Dc (for example, clockwise); (2) a supply duct 31 that discharges and supplies the mixed air 3 containing the pulp fibers 2 (corresponding to air) from a supply opening 31a to an outer circumferential face 20a of the drum 20, the supply opening 31a being arranged at a predetermined position (corresponding to a first position) in the circumferential direction Dc of the drum 20; and (3) a suction conveyor 41 that is arranged downstream of circumferential direction Dc from the supply duct 31 and that sucks the absorbent body 1 to remove it from a mold 21 on the outer face 20a of the drum 20 and transports the absorbent body 1.

In the following description, the circumferential direction Dc of the rotatable drum 20 is referred to as merely "the circumferential direction", and a direction along the horizontal axis C20 of the rotatable drum 20 (in FIG. 1, a direction perpendicular to the paper surface) is referred to as a "width direction" or "right-to-left direction".

The rotatable drum 20 is a substantially cylindrical object, and, on its outer circumferential face 20a, there are provided the molds 21 that is of a depression corresponding to a shape of the absorbent body 1 to be formed, the molds 21 being provided intermittently at a predetermined pitch in the circumferential direction Dc. As a bottom of each mold 21, an air-permeable member 50 is provided, and the inside of the mold 21 communicates with the inside of the rotatable drum 20 air-permeably through air-through holes 51 of the air-permeable member 50.

A cylindrical wall 22a is provided inside the rotatable drum 20 coaxially with the rotatable drum 20; thereby, a substantially closed space S having a doughnut-shape is formed on an inner circumferential face of the rotatable drum 20. Further, this substantially closed space S is divided by a plurality of walls 22b into a plurality of zones along the circumferential direction Dc; for example, a first zone Z1 shown in FIG. 1 which is maintained at negative pressure lower than the external pressure, and a second zone Z2 downstream therefrom which is maintained at a pressure slightly higher than or equal to the external pressure. Corresponding to this first zone Z1, the supply opening 31a of the supply duct 31 is provided, and, corresponding to the second zone Z2, the suction conveyor 41 is provided.

According to this fiber depositing apparatus 10, the absorbent body 1 is formed as follows. With rotation of the rotatable drum 20, the mold 21 passes the supply duct 31. The mixed air 3 are discharged from and supplied by the supply opening 31a, and substantially only the air component thereof is drawn into the air-permeable member 50 on the bottom of the mold 21. As a result thereof, the pulp fibers 2 contained in the mixed air 3 are deposited onto the same air-permeable member 50. When the mold 21 passes the supply opening 31a and reaches a position opposite the suction conveyor 41, the pulp fibers 2 in the mold 21 are sucked outwardly by suction of the suction conveyor 41 and are removed successively from the mold 21. Thereafter, the pulp fibers are transported by the suction conveyor 41 as an absorbent body 1.

Incidentally, it is possible that a polymer injection pipe (not shown) is provided in the supply duct 31 to discharge superabsorbent polymer from an opening of the polymer injection pipe towards the outer circumferential face 20a. In this case, this superabsorbent polymer also serves as a liquid absorbent material .

### <<<Configuration of Rotatable Drum 20 and Mold 21>>>

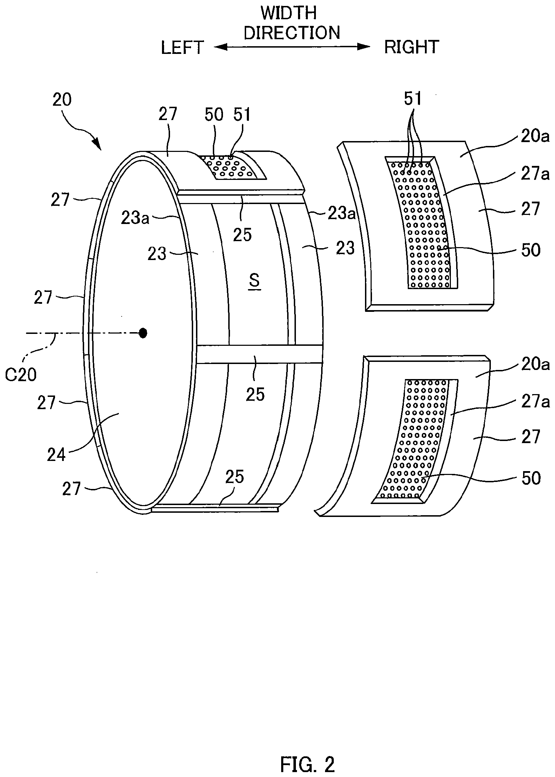

FIG. 2 is of exploded perspective view showing the rotatable drum 20.

The rotatable drum 20 provides a pair of ring members 23, 23 that are arranged symmetrically relative to a center in the width direction;
a plurality of connecting plates 25 that connect the ring members 23, 23 with maintaining a distance between these ring members 23, 23; and
a plurality of mold plates 27 (corresponding to a mold member) that extend between the connecting plates 25, 25 and serve as the outer circumferential face 20a of the rotatable drum 20, the connecting plates 25, 25 being adjacent to each other at a suitable pitch in the circumferential direction Dc.

The pair of ring members 23, 23 are perfectly circular rings and both have the same shape. On each of edges 23a positioned on the sides on which the ring members 23, 23 do not oppose in the width direction of the rotatable drum 20, a circular wall 24 having a similar shape to the perfectly circular ring member 23 is provided, and the wall closes the drum. As a result, the substantially closed space S mentioned above is formed inside the ring members 23, 23.

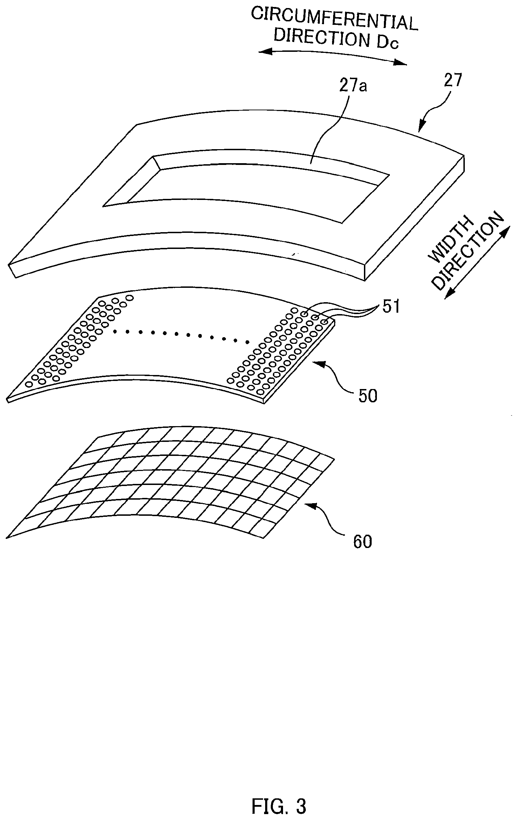

The mold plate 27 is an arcuate plate 27 whose length is the length of the outer circumferential face 20a of the rotatable drum 20 divided equally by the number of the molds 21 to be provided (seven in this example). On the planar center of the arcuate plate 27, an opening 27a is provided having a shape corresponding to a shape in which the absorbent body 1 is to be formed. As shown in the exploded perspective view of the mold plate 27 in FIG. 3, the opening 27a is covered with the air-permeable member 50 inside the rotatable drum 20, and the air-permeable member 50 serves as a bottom of the mold 21 on which the pulp fibers 2 are to be deposited. The air-permeable member 50 is fixed by welding or the like continuously or intermittently on the entire edge of the opening 27a. In this example, a single opening 27a serving as the mold 21 is provided on each of the mold plates 27, but this invention is not limited thereto. For example, two or more openings 27a can be provided on the each the mold plate 27.

As shown in FIG. 2, a connecting plate 25 is a plate elongated in the width direction of the rotatable drum 20, for example. The connecting plates 25 are arranged such that these plates are not superimposed on any part of the openings 27a of the mold plate 27. As a result, air-permeability of the openings 27a is maintained in a good state that there is no member to impair the air-permeability except for the air-permeable member 50 and a reinforcing member 60 described later.

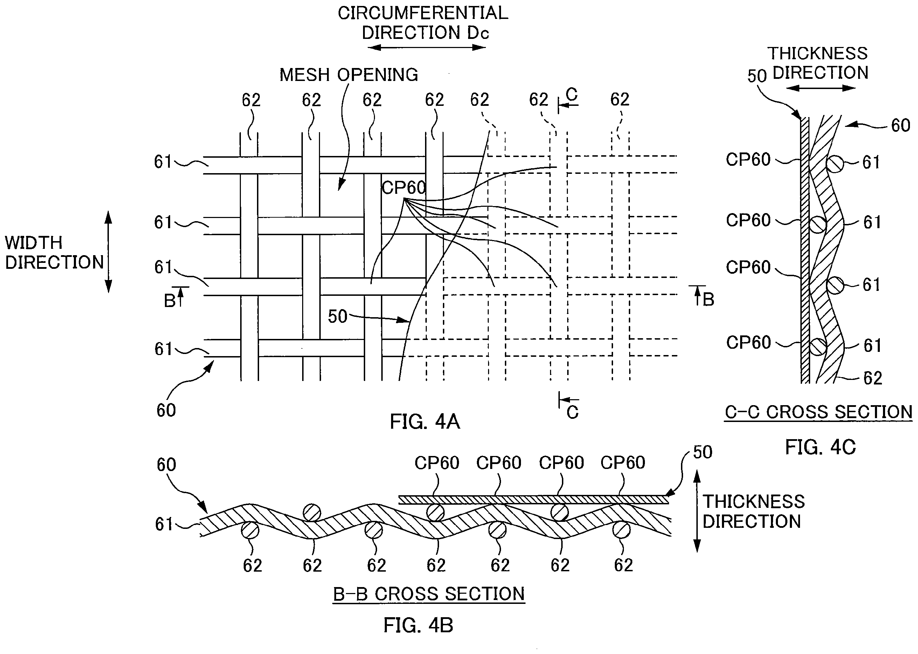

FIGS. 4A to 4C shows an explanatory diagram of the air-permeable member 50 according to the invention. FIG. 4A is an enlarged plan view showing the air-permeable member 50 that is partially omitted, and FIGS. 4B and 4C are respectively the B-B cross-sectional view and the C-C cross-sectional view in FIG. 4A. In FIGS. 4A to 4C, the air-through holes 51 of the air-permeable member 50 are omitted in order to avoid complications regarding the diagrams.

The air-permeable member 50 has a plurality of the air-through holes 51 extending through the member 50 along the thickness direction (not shown in FIGS. 4A to 4C). These air-through holes 51 allow air to pass in the thickness direction, and regulate passage of the pulp fibers 2. As an example, the air-permeable member 50, a woven wire cloth described later or the like, is provided. A specific example of the air-permeable member 50 will be described later.

However, rigidity of the air-permeable member 50 alone is low and bending deformation becomes more likely to occur by suction forces that act in the directions opposite to each other while depositing or removing of the aforementioned absorbent body 1. As a result thereof, there is a possibility that fatigue failure occurs to the air-permeable member 50. Therefore, on one surface of the air-permeable member 50 (a surface opposite to the surface on which the absorbent body 1 is deposited while being mounted on the rotatable drum 20), the reinforcing member 60 that reinforces the air-permeable member 50 is superimposed and is joined across substantially entire areas of that surface.

As the reinforcing member 60, a mesh formed by connecting a first wire 61 and a second wire 62 that intersect, at intersection points CP60 thereof, is employed. The reason because a mesh is employed is not to impair air-permeability of the air-permeable member 50. The first wire 61 and the second wire 62 are woven. Here, the term "be woven" means a state in which the first wire 61 and the second wire 62 are engaged with switching positions of these wires 61, 62 in the thickness direction at each one or more of the intersection points CP60. By being woven, the first wire 61 and the second wire 62 are firmly connected at the intersection points CP60.

This joining of the reinforcing member 60 to the air-permeable member 50 is performed at the intersection points CP60 of the first wire 61 and second wire 62. This is because the intersection points CP60 are a portion having relatively higher rigidity than a portion formed by the first wire 61 or the second wire 62 alone, due to connection of these wires. Therefore, the intersection points CP60 can sufficiently resist suction force that acts on the air-permeable member 50 while depositing or removing the absorbent body 1, so that deformation of the air-permeable member 50 is suppressed surely. In addition, since the air-permeable member 50 and the reinforcing member 60 are joined and integrated at the intersection points CP60, the members substantially integrally deform. Therefore, the distance between the air-permeable member 50 and the reinforcing member 60 is more likely to be held constant. As a result, even if a pulp fiber 2 is sandwiched between the air-permeable member 50 and the reinforcing member 60, the pulp fiber 2 is likely to be released and clogging of the air-through holes 51 of the air-permeable member 50 or of the mesh opening of the reinforcing member 60 is effectively prevented.

It is most desirable that the reinforcing member 60 is joined to the air-permeable member 50 at all of the intersection points CP60 that are positioned in the opening 27a of the mold plate 27. However, equal to or more than half of the intersection points CP60 is acceptable, and further equal to or more than two intersection points is also acceptable. Furthermore, one intersection point achieves the reinforcement effect.

With respect to the planar size of the air-through hole 51 (a mesh opening in the case of a woven wire cloth) of the air-permeable member 50, any suitable size can be selected depending on size of the liquid absorbent material such as the pulp fiber 2, superabsorbent polymer or the like. However, it is preferred that the opening area is in the range of 0.01 to 1 mm². This is because, if the opening area is less than 0.01mm², the pressure loss increases, the suction power that is needed while depositing and removing increases, and clogging of the pulp fiber 2 is more likely to occur. On the other hand, if the opening area is larger than 1mm², the pulp fiber 2 becomes less likely to be caught.

Further, the opening area of the mesh opening of the reinforcing member 60 is desirably greater than opening area of the air-through hole 51 of the air-permeable member 50. This makes it possible to effectively suppress deterioration of air-permeability of the air-permeable member 50 due to providing the reinforcing member 60.

The air-permeable member 50 and the reinforcing member 60 will be described below in detail with showing their examples.

### <<< First Embodiment >>>

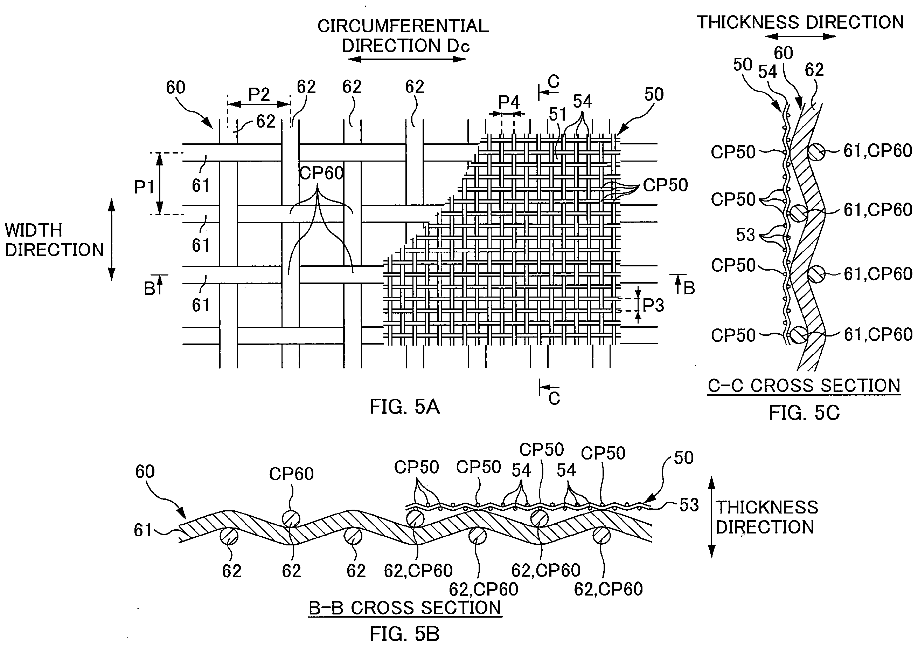

FIGS. 5A to 5C shows the first embodiment of the air-permeable member 50 not being part of the invention and the reinforcing member 60. FIG. 5A is a plan view showing the air-permeable member 50 that is partially omitted herein, FIGS. 5B and 5C are respectively a B-B cross-sectional
view and a C-C cross-sectional view in FIG. 5A.

The air-permeable member 50 according to the first embodiment is a mesh formed by weaving a third wire 53 and a fourth wire 54 that intersect; more specifically, a plain woven wire cloth. The mesh opening thereof serves as the foregoing air-through hole 51. On the other hand, the reinforcing member 60 is plain woven wire cloth which is formed by weaving the first wire 61 and second wire 62 whose wire diameters are larger than those of the third and fourth wire 53, 54. In this example, both of the woven wire cloth is made of metal wire such as stainless steel (SUS304 in this example), but non-metal such as resin or the like can be employed if the plate has suitable rigidity and stiffness. In terms of joining capability, it is desirable that materials of the air-permeable member 50 and the reinforcing member 60 are the same.

The plain weave is a weaving process by crossing the warp wire and the weft wire over and under each other one by one. In the air-permeable member 50, the third wire 53 is arranged as warp wire along the circumferential direction Dc of the rotatable drum 20, and the fourth wire 54 is arranged as weft wire along the width direction of the drum 20; these warp wire and weft wire are orthogonal at intersection points CP50. On the other hand, in the reinforcing member 60, the first wire 61 is arranged as warp wire, and the second wire 62 is arranged as weft wire; these warp wire and weft wire are orthogonal at the intersection points CP60.

In the air-permeable member 50, the third wire 53 is arranged at the third pitch P3 in the longitudinal direction of the fourth wire 54, and the fourth wire 54 is arranged at the fourth pitch P4 in the longitudinal direction of the third wire 53. In addition, these third pitch P3 and fourth pitch P4 are the same distance. As a result, a mesh opening 51 is square in shape. In the reinforcing member 60, the first wire 61 is arranged at the first pitch P1 in the longitudinal direction of the second wire 62, and the second wire 62 is arranged at second pitch P2 in the longitudinal direction of the first wire 61. In addition, these first pitch P1 and second pitch P2 are the same distance. As a result, the mesh opening is square in shape.

Combinations of the air-permeable member 50 and the reinforcing member 60, which is determined depending on combinations of the foregoing first pitch P1 to fourth pitch P4 and the wire diameters, are a myriad of choices. However, basic conditions to be satisfied is the foregoing two conditions (the opening area of the mesh opening 51 of the air-permeable member 50, and the relationship in opening area between the mesh opening of the reinforcing member 60 and the mesh opening 51 of the air-permeable member 50). With respect to combinations satisfying these two conditions, the examples below are provided

The first example is the following combination: the air-permeable member 50 in which wire diameter is 0.27mm, the third pitch P3 and fourth pitch P4 is pitch corresponding to 40 per inch, and opening ratio (the ratio of unit area of the air-permeable member 50 to opening area of the air-through hole 51(mesh opening)) is 33%; and the reinforcing member 60 in which wire diameter is 0.9mm, the first pitch P1 and second pitch P2 is pitch corresponding to 4 per inch, and opening ratio(the ratio of a unit area of the reinforcing member 60 to opening area of the mesh opening) is 73.7%.

The second example is the following combination: the air-permeable member 50 in which wire diameter is 0.12mm, the third pitch P3 and fourth pitch P4 is pitch corresponding to 80 per inch, and opening ratio is 38.7%; and the reinforcing member 60 in which wire diameter is 0.9mm, the first pitch P1 and second pitch P2 is pitch corresponding to 4 per inch, and opening ratio is 73.7%.

It is desirable that, with respect to the arrangement pitchs of warp wires and weft wires, the following relationship is satisfied between the reinforcing member 60 and the air-permeable member 50.

In other words, it is desirable to satisfy the relationship "the first pitch P1, at which the warp wires of the reinforcing member 60 are arranged, is integer multiple (more than or equal to 2 times) of the third pitch P3, at which the warp wires of the air-permeable member 50 are arranged; and the second pitch P2, at which the weft wires of the reinforcing member 60 are arranged, is integer multiple (more than or equal to 2 times) of the fourth pitch P4, at which the weft wires of the air-permeable member 50 are arranged". In the example of FIGS. 5A to 5C, the aforementioned magnification is 5 times.

According to this configuration, the intersection points CP50 of the air-permeable member 50 can be arranged to ensure corresponding to all the intersection points CP60 of the reinforcing member 60, which are in the opening 27a of the mold plate 27. This makes it possible to match up and to join all intersection points CP60 of the reinforcing member 60 and the intersection points CP50 of the air-permeable member 50. Because of joints that have high rigidity and are formed by matching up the intersection points CP60 and CP50, deformation of the air-permeable member 50 is effectively suppressed more surely across wider area.

It is desirable that the air-permeable member 50 related to the third wire 53 and fourth wire 54 are joined at the intersection points CP50 of these wires 53, 54. This enables rigidity of the air-permeable member 50 to increase. Deformation of the air-permeable member 50 is suppressed, and fatigue failure becomes less likely to occur. In addition, because deformation of the mesh opening 51 is also suppressed, the pulp fiber 2 is also effectively prevented from being caught by and clogging the mesh opening 51 due to deformation of the opening 51. This can be said with respect to the reinforcing member 60. In other words, it is desirable that the first wire 61 and second wire 62 of the reinforcing member 60 are joined at the intersection points CP60 of these wires 61, 62.

As joining method, diffusion welding is provided for example. Diffusion welding means "a method in which a joint can be achieved in intimate contact of the workpieces at a temperature below the melting point of the workpieces under such pressure that plastic deformation can be suppressed as much as possible, by using diffusion of atoms across the interface" (see Japanese Industrial Standard JIS Z3001-2, 22702).

For example, in case where the material of the air-permeable member 50 and reinforcing member 60 are both SUS304, the air-permeable member 50 and reinforcing member 60 are heated with being superimposed on each other, for example, within the range of 800°C to 1200°C not more than melting point of SUS304. Concurrently, both of the air-permeable member 50 and reinforcing member 60 are caught and pressed by a predetermined pressing member. As a result, the intersection points CP60 of the reinforcing member 60 are joined to the air-permeable member 50, the first wire 61 and the second wire 62 of the reinforcing member 60 are joined , and the third wire 53 and the fourth wire 54 of the air-permeable member 50 are joined.

According to diffusion welding, joining can be performed without brazing filler metal such as solder, filler rod or the like. This makes it possible to prevent lump of a brazing filler metal from remaining in the mesh or the like and to maintain good air-permeability of the air-permeable member 50, the reinforcing member 60 or the like.

As described below, if pressing is to be performed after diffusion welding, it is preferable that, in order to increase the workability (plastic deformation capacity) in processing, the foregoing diffusion welding is performed under inert gas atmosphere such as algon gas, nitrogen gas.

The mold plate 27 of which these air-permeable member 50 and reinforcing member 60 are disposed is arcuate as shown in FIG. 2. Therefore, the air-permeable member 50 and the reinforcing member 60 are required to be bent such that the bending fits the curvature of the inner circumferential face of the mold plate 27. Further, if the absorbent body 1 is required to be formed in a three dimensional shape having a localized region with a different weight of fibers per unit area, a depression or a protrusion is required to be formed in a shape corresponding to the three dimensional shape, on the air-permeable member 50 and the reinforcing member 60.

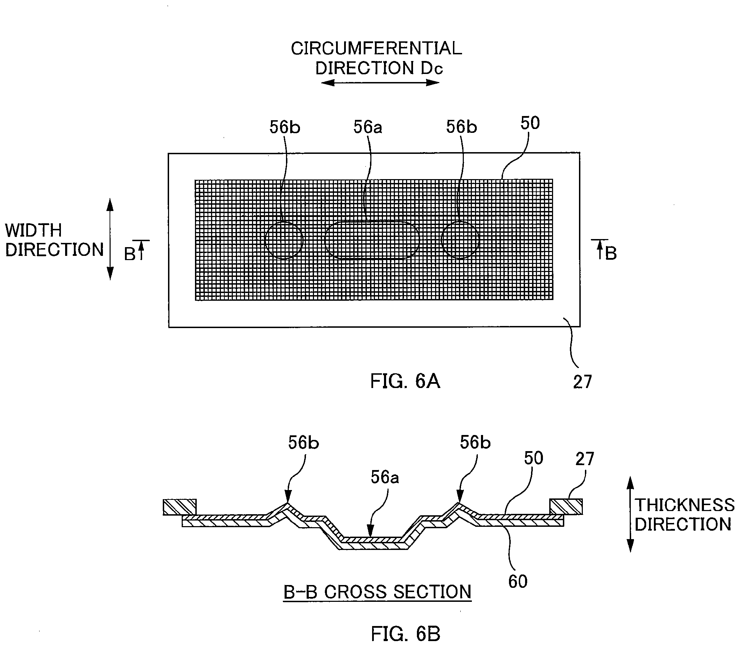

This bending and forming of a depression or a protrusion is performed, after integrating the air-permeable member 50 and the reinforcing member 60 by diffusion welding, by pressing while the members being integrated. In examples shown in the plan view of FIG. 6A and the longitudinal sectional view of FIG. 6B, target 3D shape of the air-permeable member 50 and the reinforcing member 60 has a depression 56a and a protrusion 56b extending in the thickness direction. Therefore, as a press mold, a male press mold and a female press mold are employed that have a curving shape as a whole and that provide a depression or a protrusion corresponding to the depression 56a and the protrusion 56b. In addition, by sandwiching and pressing the air-permeable member 50 and reinforcing member 60 between these male and female press molds, the air-permeable member 50 and reinforcing member 60 undergo plastic deformation, and the curving shape or the depression 56a and the protrusion 56b are formed thereon. In order to increase plastic deformation capacity of this case, in other words, the ability to deform the air-permeable member 50 or the reinforcing member 60 without failure, it is necessary to perform the aforementioned diffusion welding under inert gas atmosphere.

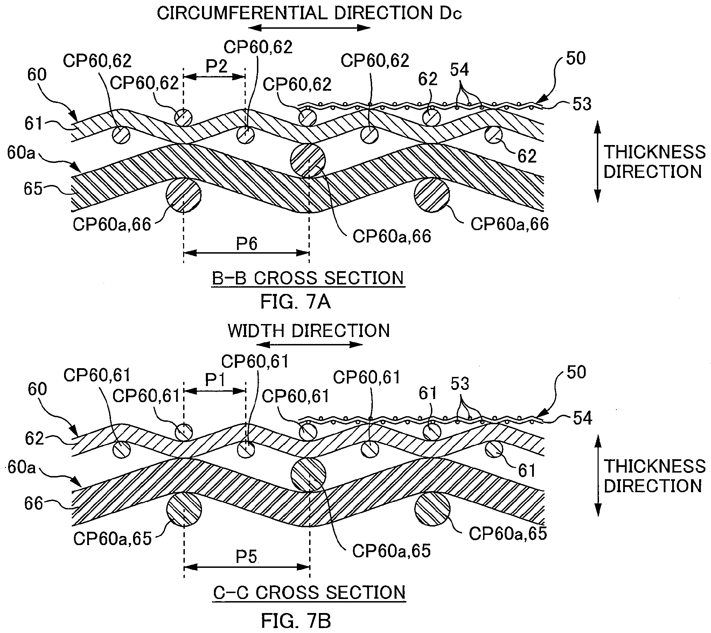

FIGS. 7A and 7B are explanatory diagrams showing the modified example of the first embodiment, and longitudinal sectional views respectively corresponding to the B-B cross section and the C-C cross section in FIG. 5A. In the first embodiment, reinforcement by one layer configuration is described in which a single reinforcing member 60 is superimposed on the air-permeable member 50. This modified example differs therefrom in that reinforcement by two layer configuration is employed by superimposing two reinforcing members 60 as an example of a plurality of the reinforcing members, but the other points are generally the same. Therefore, the same sign is used for the same configurations, and description thereof is omitted.

As shown in FIGS. 7A and. 7B, in this modified example, in addition to the reinforcing member 60 that is joined to the air-permeable member 50 (hereinafter referred to as a first reinforcing member 60), the reinforcing member 60a (hereinafter referred to as a second reinforcing member) is superimposed thereon, and the members are joined and integrated. As a result, rigidity of the air-permeable member 50 further increases.

The second reinforcing member 60a is also a plain weave wire cloth, for example. More specifically, the second reinforcing member 60a have a fifth wire 65 and a sixth wire 66 that intersect, the wire diameters thereof are larger than those of the first and second wire 61, 62 of the first reinforcing member 60. The fifth wire 65 is arranged as warp wire along the circumferential direction Dc of the rotatable drum 20, and the sixth wire 66 is arranged as weft wire along the width direction of the drum 20; these warp wire and weft wire are orthogonal at intersection points CP60a. In addition, the fifth wire 65 is arranged at a fifth pitch P5 in the longitudinal direction of the sixth wire 66, and the sixth wire is arranged at a sixth pitch P6 in the longitudinal direction of the fifth wire. In addition, these fifth pitch P5 and sixth pitch P6 are the same distance. As a result, a mesh opening is square in shape.

The fifth pitch P5, at which warp wire of the second reinforcing member 60a is arranged, is set to integer multiple (equal to or more than 2 times; 2 times herein) of the first pitch P1, at which warp wire of the first reinforcing member 60 is arranged. The sixth pitch P6, at which weft wire of the second reinforcing member 60a is arranged, is set to integer multiple (equal to or more than 2 times; 2 times herein) of the second pitch P2, at which weft wire of the first reinforcing member 60 is arranged.

Therefore, the intersection points CP60 of the first reinforcing member 60 can be arranged surely corresponding to all of the intersection points CP60a of the second reinforcing member 60a. In other words, it is possible to match up and join all intersection points CP60a of the second reinforcing member 60a and the intersection points CP60 of the first reinforcing member 60. This enables reinforcement effect to further improve.

It is possible that another reinforcing member satisfying the aforementioned pitch relationship is provided to the second reinforcing member 60a and these members are joined and integrated to form three layer configuration having the reinforcing members. Furthermore, it is possible that another reinforcing member satisfying the pitch relationship is successively superimposed thereon to form multi layer configuration having four or more reinforcing members.

### <<< Second embodiment >>>

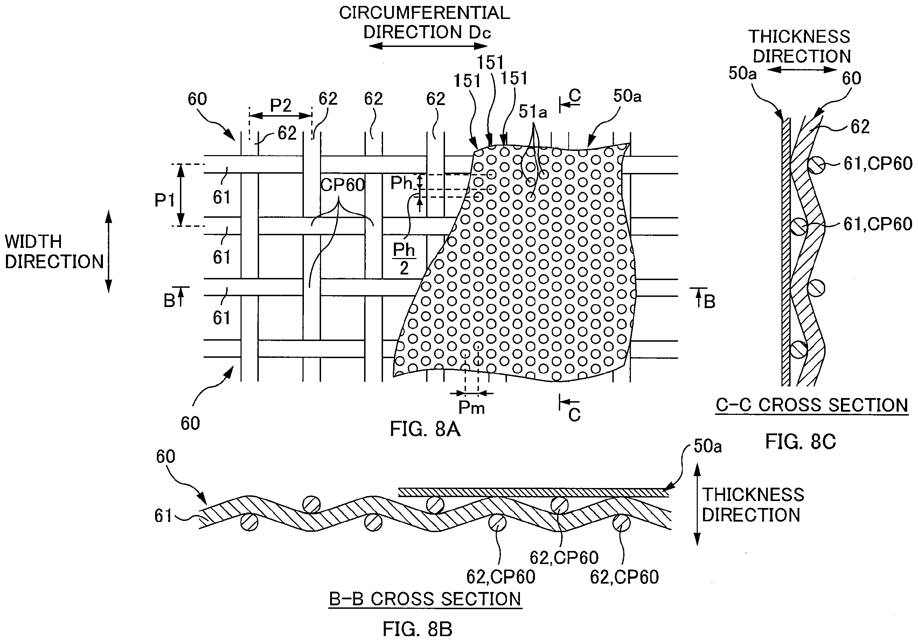

FIGS. 8A to 8C shows the second embodiment of an air-permeable member 50a and the reinforcing member 60. FIG. 8A is a plan view showing the air-permeable member 50a that is partially omitted herein, FIGS. 8B and 8C are respectively a B-B cross-sectional view and a C-C cross-sectional view in FIG. 8A. In FIGS. 8B and 8C, air-through holes 51a of the air-permeable member 50a are omitted in order to avoid complications regarding the diagrams.

In the first embodiment, woven wire cloth is employed as an air-permeable member 50. This second embodiment differs therefrom in that plate member is employed, but the other points are generally the same. Therefore, the same sign is used for the same configurations, and description thereof is omitted.

The air-permeable member 50a is such metal plate having a predetermined thickness (0.3 mm in thickness herein) as stainless steel plate (SUS304 in this example)or the like Non-metal plate such as a resin plate or the like can be employed if the plate has suitable rigidity and stiffness. However, in terms of joining capability, it is desirable that the material of the air-permeable member 50a and the material of the reinforcing member 60 are the same.

As shown in 8A, across substantially entire areas of the air-permeable member 50a, through holes 51a are formed as the air-through hole 51a along the thickness direction in a predetermined arrangement pattern. The air-through hole 51a are formed, for example, in perfectly circular shape having a diameter of 0.3 mm, and the opening ratio thereof is 32.43%, for example. The arrangement pattern is zig zag pattern, for example. In other words, air-through-hole lines 151 are provided by lining up a plurality of the air-through holes 51a at a predetermined pitch Ph (0.5mm in this example) along the width direction of the rotatable drum 20, and the basic pattern thereof is that the position of each air-through-hole line 151 is shifted half pitch (=Ph/2) in the width direction with respect to the position of the adjacent air-through-hole line 151 in the circumferential direction Dc of the rotatable drum 20. However, in this example, in addition thereto, a pitch Pm at which the air-through-hole lines 151 are arranged in the circumferential direction Dc is set to a pitch defined by the formula: Pm = 2 × Ph × cos30°. This allows to make equal distances between each of the neighboring air-through holes 51a, 51a with respect to all of the air-through holes 51a. It can be said that a unit of the arrangement pattern in this case is a pattern that each vertex of an eqilateral triangle is a position of each air-through hole 51a, and that the arrangement pattern is formed by repeating the unit across substantially entire areas. The length of the side of the eqilateral triangle is equal to the aforementioned Ph, for example 0.5mm.

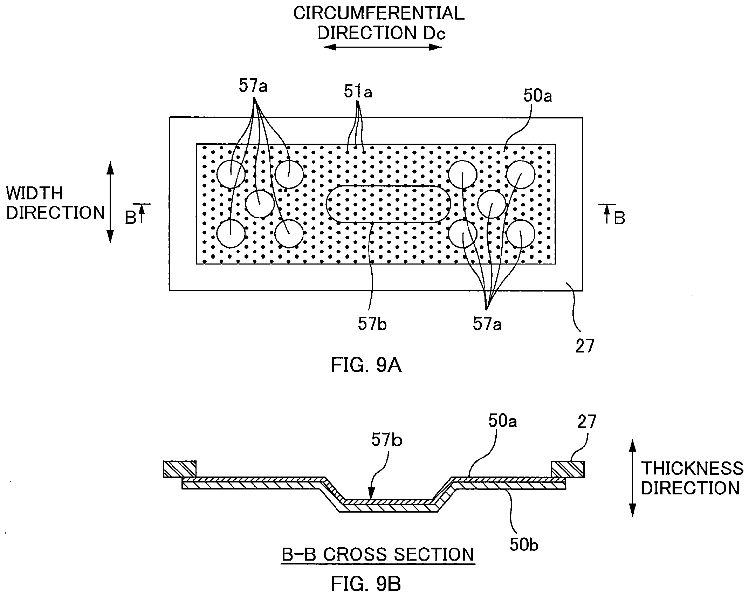

These air-through holes 51a are formed completely through the plate by etching, for example. More specifically, on the plate of the air-permeable member 50a, regions corresponding to regions where air-through holes 51a are not to be formed are masked by a corrosion inhibitor. Thereafter, regions corresponding to the air-through holes 51a alone are corroded by a suitable chemical agent, and the air-through holes 51a are formed completely through the plate. As shown in the plan view of FIG. 9A and the longitudinal sectional view of FIG. 9B, in order to form a localized portion having a small weight of fibers per unit area in an absorbent body 1, it is necessary to mask a corresponding region 57a to the portion by a corrosion inhibitor, as shown in FIG. 9A As a result, a low density region 57a is formed in which the density of the air-through hole 51a is lower than in surrounding region, and the weight of the pulp fiber 2 per unit area in the low density region 57a can be small.

On the other hand, as shown in FIGS. 8A to 8C, the reinforcing member 60 is a woven wire cloth in similar to the first embodiment. As an example thereof, as mentioned above, a woven wire cloth is provided in which wire diameter is 0.9mm, the first pitch P1 and second pitch P2 is pitch corresponding to 4 per inch, and opening ratio is 73.7%.

Joining of the air-permeable member 50a and the reinforcing member 60, identically to the foregoing first embodiment, is performed at the intersection points CP60 of the first wire 61 and second wire 62 of the reinforcing member 60, and diffusion welding is provided as a method of joining. Further, in the case of bending the air-permeable member 50a and the reinforcing member 60 or forming the depression 57b and protrusion, the pressing in the similar procedure to the first embodiment is performed. In other words, after integrating the air-permeable member 50a and reinforcing member 60 by diffusion welding, pressing is performed with the members being integrated.

### === Other embodiments ===

The embodiments of the invention have been described above, but the invention should not be limited to these embodiments, and modifications described below are possible which belong to the invention as long as they fall under claim 1.

In the foregoing first embodiment and second embodiment, as the reinforcing member 60, the woven wire cloth in which the first wire 61 and the second wire 62 are orthogonal is described. However, an angle at which the first wire 61 and the
second wire 62 intersect is not limited to 90 degrees. In other words, the angle can be set at an angle other than 90 degrees such that the mesh opening is parallelogram, rhombus or the like in shape.

In the foregoing first embodiment, as an air-permeable member 50, a woven wire cloth in which the third wire 53 and the fourth wire 54 are orthogonal is described. However, an angle at which the third wire 53 and the fourth wire 54 intersect is not limited to 90 degrees. In other words, the angle can be set at an angle other than 90 degrees such that the mesh opening is parallelogram, rhombus or the like in shape.

In the modified example of the foregoing first embodiment, as the second reinforcing member 60a, the woven wire cloth in which the fifth wire 65 and the sixth wire 66 are orthogonal is described. However, an angle at which the fifth wire 65 and the sixth wire 66 intersect is not limited to 90 degrees. In other words, the angle can be set at an angle other than 90 degrees such that the mesh opening is parallelogram, rhombus or the like in shape.

In the foregoing first embodiment and second embodiment, the first pitch P1 of the first wire 61 and the second pitch P2 of the second wire 62 are the same. However, these pitches can be different from each other.

In the foregoing first embodiment, the third pitch P3 of the third wire 53 and the fourth pitch P4 of the fourth wire 54 are the same. However, these pitches can be different from each other.

In the foregoing first embodiment and second embodiment, the first wire 61 and the second wire 62 have the same wire diameter, but this invention is not limited thereto. These wires can have different diameter.

In the foregoing first embodiment, the third wire 53 and the fourth wire 54 have the same wire diameter, but this invention is not limited thereto. These wires can have different diameter.

In the foregoing first embodiment, a plain weave wire cloth is employed as the air-permeable member 50 and the reinforcing member 60, this invention is not limited thereto. For example, twill weave wire cloth can be used for both of them. Furthermore, it is possible that the plain weave wire cloth is employed for either one of the air-permeable member 50 and the reinforcing member 60 and the twill weave wire cloth is employed for the other one. However, in terms of corresponding the intersection points CP60 to the intersection points CP50, it is preferable that a weaving of the reinforcing member 60 is aligned with a weaving of the air-permeable member 50, the longitudinal directions of the first wire 61 and the third wire 53 are aligned with each other, and the longitudinal directions of the second wire 61 and the fourth wire 54 are aligned with each other.

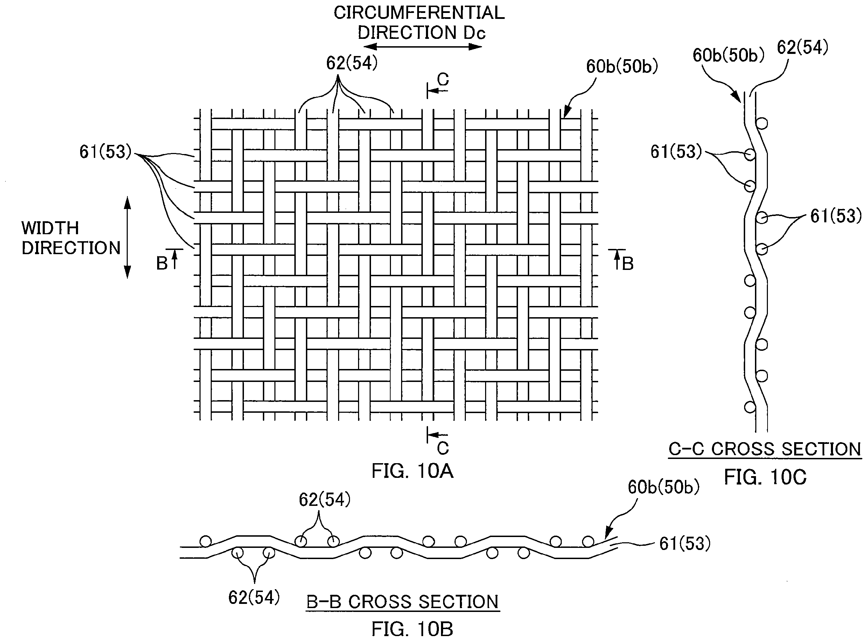

The twill weave is a weaving process by lining up and crossing two or more of the warp wires 61(53) and the weft wires 62(54) continuously regularly over and under each other, as shown in FIGS. 10A to 10C.

Furthermore, with respect to the reinforcing member 60, the first wire 61 and the second wire 62 can be connected at the intersection points CP60; in other words, a configuration is not limited to a configuration in which the wires are woven such as the foregoing plain weave wire cloth, twill weave wire cloth or the like.

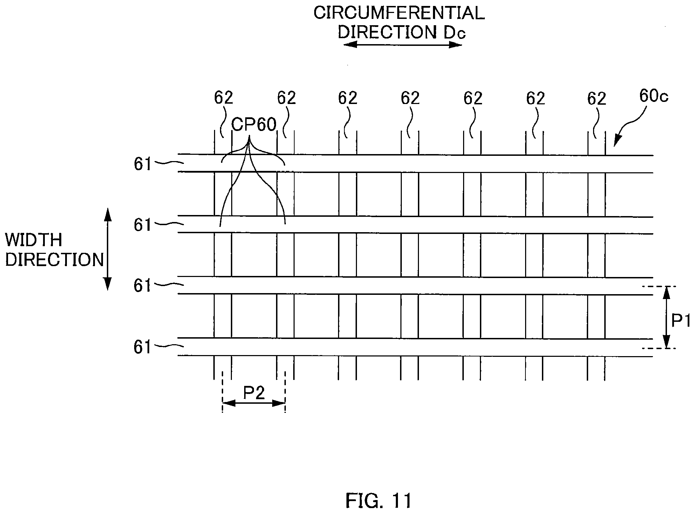

For example, as shown in the plan view of FIG. 11, the positional relationship of the first wire 61 and the second wire 62 in the thickness direction (in FIG. 11, a direction perpendicular to the paper surface) is not required to be switched. In other words, the reinforcing member 60c is also acceptable having a configuration in which all of the first wires 61 are positioned on the one side to the thickness direction, all of the second wires 62 are positioned on the other side, and the wires 61, 62 are joined at the intersection points CP60. However, a woven configuration is more preferred since the first wire 61 and second wire 62 are firmly connected at the intersection points CP60.

In the foregoing embodiments, the reinforcing member 60 is arranged on one of surfaces of the air-permeable member 50 (or 50a). However, it is possible that the reinforcing member 60 are arranged on both of the surfaces of the air-permeable member 50 (or 50a) and the air-permeable member 50 (or 50a) is sandwiched between and reinforced by the pair of reinforcing members 60, 60. More specifically, the reinforcing member 60 can be superimposed and arranged on the one surface of the air-permeable member 50 (or 50a), and the member 60 can be joined to the air-permeable member 50 (or 50a) at the intersection points CP60 of the first wire 61 and second wire 62 of the reinforcing member 60. In addition, another reinforcing member 60 can be superimposed and arranged on the other surface of the air-permeable member 50 (or 50a), the member 60 can be joined to the air-permeable member 50 (or 50a) at the intersection points CP60 of the first wire 61 and second wire 62 of this reinforcing member 60. In this case, under suction forces that act in both of opposite directions along the thickness direction, a tension load that acts on the joined portion at the intersection points CP60 is reduced. Therefore, joining at the intersection points CP60 is less likely to break.

In the foregoing second embodiment, as an example of the air-permeable member 50a, there is provided the plate member having the air-through holes 51a that is of perfect circle in shape and is arranged in zig zag pattern and whose opening ratio is 32.43%, but this invention is not limited thereto. For example, the air-through holes 51a can be ellipse, polygon or the like in shape, the arrangement pattern thereof can be grid pattern (squared pattern)or the like, and the opening ratio thereof can be set at a percentage other than mentioned above.

In the description of the foregoing embodiments, the reinforcing member 60 is joined to the air-permeable member 50 at the intersection points CP60. This means "the members are joined at least at the intersection points CP60, and therefore includes a concept that the members are joined at a portion that surrounds the intersection points CP60 and at an area at which the reinforcing member 60 and air-permeable member 50 contact.

### Reference Signs List

1 absorbent body, 2 pulp fiber (liquid absorbent material), 3 mixed air (air),
10 fiber depositing apparatus (manufacturing apparatus of absorbent body),
20 rotatable drum, 20a outer circumferential face, 21 mold,
22a cylindrical wall, 22b wall,
23 ring member, 23a edge, 24 circular wall,
25 connecting plate,
27 mold plate (mold member), 27a opening,
31 supply duct, 31a supply opening,
41 suction conveyor,
50 air-permeable member, 50a air-permeable member,
51 mesh opening (air-through hole), 51a air-through hole,
53 third wire, 54 fourth wire,
56a depression, 56b protrusion,
57a low density region, 57b depression,
60 reinforcing member, 60a reinforcing member, 60c reinforcing member,
61 first wire, 62 second wire,
65 fifth wire, 66 sixth wire,
151 air-through-hole line,
Z1 first zone, Z2 second zone,
C20 axis,
CP50 intersection point, CP60 intersection point, CP60a intersection point,
S substantially closed space

## Claims

1. A manufacturing apparatus for an absorbent body (1) associated with an absorbent article, the manufacturing apparatus manufacturing the absorbent body using a procedure in which a liquid absorbent material is deposited on an air-permeable member (50a) by passing air that contains the liquid absorbent material in a thickness direction of the air-permeable member (50a), the air-permeable member (50a) covering an opening of a mold member, comprising:
a reinforcing member (60) for reinforcing the air-permeable member (50a) by being superimposed on the air-permeable member (50a) in the thickness direction,
the reinforcing member (60) being a mesh formed by connecting a first wire (61) and a second wire (62) that intersect, at an intersection point of these wires, and
the reinforcing member (60) being joined to the air-permeable member (50a) at the intersection point, wherein
a main body of the air-permeable member (50a) is a plate member, and
an air-through hole (51a) is formed along the thickness direction completely through the plate member,
the manufacturing apparatus further comprises a cylindrical rotatable drum (20) that rotates continuously in a circumferential direction,
the mold member forms an outer circumferential face of the rotatable drum (20), air-permeability of the opening of the mold member being maintained in a state that there is no member to impair the air-permeability except for the air-permeable member (50a) and the reinforcing member (60),
an inner space and an outer space of the rotatable drum (20) are in air-permeably communication through the air-permeable member (50a).

2. A manufacturing apparatus for an absorbent body according to claim 1, wherein the first wire (61) and the second wire (62) are connected at the intersection point by weaving.

3. A manufacturing apparatus for an absorbent body according to claim 2, wherein the first wire (61) and the second wire (62) are joined at the intersection point.

4. A manufacturing apparatus for an absorbent body according to claim 1, wherein the plate member has a low density region in which a density of the air-through hole is lower than a surrounding region, within the opening.

5. A manufacturing apparatus for an absorbent body according to any one of claims 1 to 4, wherein the joining is performed by diffusion welding under inert gas atmosphere.

6. A manufacturing apparatus for an absorbent body according to any one of claims 1 to 5, wherein
the manufacturing apparatus (10) further comprises
a supply duct that supplies the air towards the outer circumferential face of the rotatable drum (20) from outside is provided at a first position of the circumferential direction,
a pressure of an inner space that is of the rotatable drum (20) and corresponds to the first position is maintained at negative pressure that is lower than that of the outer space,
when the mold member passes the first position, the air in the supply duct is sucked through the air-permeable member (50a) into the inner space of the rotatable drum (20) and the absorbent body is deposited on the air-permeable member (50a),
a removal position at which the absorbent body is removed from the air-permeable member (50a) is set at a position downstream from the first position in the circumferential direction, and
a pressure of an inner space that is of the rotatable drum (20) and corresponds to the removal position is maintained at a pressure equal to or higher than that of the outer space.

7. A method for manufacturing an air-permeable member (50, 50a) that is used in the apparatus of claim 1 when forming an absorbent body (1) associated with an absorbent article by depositing a liquid absorbent material, that is reinforced by a reinforcing member (60), and that provides at least either one of a depression and a protrusion having a shape corresponding to a target shape of the absorbent body (1), wherein
a material of the air-permeable member (50, 50a) is a member regulating passage of the liquid absorbent material in a thickness direction,
a material of the reinforcing member (60) is a mesh in which a first wire (61) and a second wire (62) that intersect are connected at an intersection point of these wires, and
the method comprising:
joining and integrating the material of the air-permeable member (50, 50a) and the material of the reinforcing member (60) by diffusion welding the material of the reinforcing member to the material of the air-permeable member under inert gas atmosphere at the intersection point of the first wire (61) and the second wire (62) while superimposing in the thickness direction the material of the air-permeable member (50, 50a) and the material of the reinforcing member (60); and
forming at least either one of the depression (56a) and the protrusion (56b) by pressing the joined and integrated materials of the reinforcing member (60) and the air-permeable member (50, 50a) while sandwiching the materials between a male press mold and a female press mold.

8. A method for manufacturing an air-permeable member according to claim 7, wherein
the reinforcing member (60) is a mesh formed by weaving the first wire (61) and the second wire (62),
the air-permeable member (50) is a mesh formed by weaving a third wire (53) and a fourth wire (54) that intersect,
in joining and integrating, the first wire (61) and the second wire (62) are joined by diffusion welding at the intersection point of the first wire and the second wire, and
the third wire (53) and the fourth wire (54) are joined by diffusion welding at an intersection point of the third wire and the fourth wire.

## Patentansprüche

1. Eine Herstellungsvorrichtung für einen absorbierenden Körper (1), der mit einem absorbierenden Artikel verbunden ist, die Herstellungsvorrichtung, die den absorbierenden Körper mittels eines Verfahrens, in dem ein flüssigkeitabsorbierendes Material auf ein luftdurchlässiges Element (50a) durch Leiten von Luft, die das flüssigkeitsabsorbierende Material enthält, in einer Dickenrichtung des luftdurchlässigen Elements (50a), aufgetragen wird, herstellt, das luftdurchlässige Element (50a), das eine Öffnung eines Formteils bedeckt, umfassend:
ein Verstärkungselement (60) zum Verstärken des luftdurchlässigen Elements (50a) durch auf dem luftdurchlässigen Element (50a) in der Dickenrichtung überlagert werden,
das Verstärkungselement (60), das ein Netz ist, das durch Verbinden eines ersten Fadens (61) und eines zweiten Fadens (62), die kreuzen, an einem Schnittpunkt dieser Fäden verbunden ist, und
das Verstärkungselement (60), das an das luftdurchlässige Element (50a) an dem Schnittpunkt angefügt wird, wobei
ein Hauptkörper des luftdurchlässigen Elements (50a) ein Plattenelement ist, und
eine Luftdurchtrittsöffnung (51a) entlang der Dickenrichtung komplett durch das Plattenelement ausgebildet ist,
die Herstellungsvorrichtung ferner eine zylindrische drehbare Trommel (20) umfasst, die kontinuierlich in einer Umfangsrichtung dreht,
das Formteil eine Außenumfangsfläche der drehbaren Trommel (20) ausbildet, Luftdurchlässigkeit der Öffnung des Formteils in einem Zustand erhalten bleibt, dass es kein Element gibt die Luftdurchlässigkeit zu beeinträchtigen außer dem luftdurchlässigen Element (50a) und dem Verstärkungselement (60),
ein Innenraum und ein Außenraum der drehbaren Trommel (20) in luftdurchlässiger Verbindung durch das luftdurchlässige Element (50a) stehen.

2. Eine Herstellungsvorrichtung für einen absorbierenden Körper gemäß Anspruch 1, wobei der erste Faden (61) und der zweite Faden (62) an dem Schnittpunkt durch weben verbunden sind.

3. Eine Herstellungsvorrichtung für einen absorbierenden Körper gemäß Anspruch 2, wobei der erste Faden (61) und der zweite Faden (62) an dem Schnittpunkt verbunden sind.

4. Eine Herstellungsvorrichtung für einen absorbierenden Körper gemäß Anspruch 1, wobei das Plattenelement einen Bereich niedriger Dichte, in dem eine Dichte der Luftdurchtrittsöffnung niedriger ist als eines umgebenden Bereichs, innerhalb der Öffnung aufweist.

5. Eine Herstellungsvorrichtung für einen absorbierenden Körper gemäß einem der Ansprüche 1 bis 4, wobei das Verbinden durch Diffusionsschweißen unter Inertgasatmosphäre durchgeführt wird.

6. Eine Herstellungsvorrichtung für einen absorbierenden Körper gemäß einem der Ansprüche 1 bis 5, wobei
die Herstellungsvorrichtung (10) ferner
eine Zufuhrleitung, die die Luft in Richtung der Außenumfangsfläche der drehbaren Trommel (20) von außen zuführt, in einer ersten Position der Umfangsrichtung vorgesehen ist,
einen Druck eines Innenraums, der von der drehbaren Trommel (20) ist und zu der ersten Position korrespondiert, bei Unterdruck, der niedriger ist als der des Außenraums, gehalten wird,
wenn das Formteil die erste Position passiert, die Luft in der Zufuhrleitung durch das luftdurchlässige Element (50a) in den Innenraum der drehbaren Trommel (20) gesaugt wird und der absorbierende Körper auf dem luftdurchlässigen Element (50a) aufgetragen wird,
eine Entfernungsposition, an der der absorbierende Körper von dem luftdurchlässigen Element (50a) entfernt wird, an einer Position in Bandlaufrichtung von der ersten Position in der Umfangsrichtung gesetzt ist, und
ein Druck eines Innenraums, der von der drehbaren Trommel (20) ist und zu der Entfernungsposition korrespondiert, bei einem Druck, der gleich oder höher als dem des Außenraums ist, gehalten wird,
umfasst.

7. Ein Verfahren zur Herstellung eines luftdurchlässigen Elements (50, 50a), das in der Vorrichtung des Anspruchs 1 genutzt wird,
bei Ausbildung eines absorbierenden Körpers (1), der mit einem absorbierenden Artikel durch Auftragen eines flüssigkeitsabsorbierenden Materials verbunden ist, der durch ein Verstärkungselement (60) verstärkt ist, und der wenigstens eines von einer Vertiefung und einem Vorsprung mit einer Form, die zu einer Sollform des absorbierenden Körpers (1) korrespondiert, ausbildet, wobei
ein Material des luftdurchlässigen Elements (50, 50a) ein Element ist, das den Durchlauf des flüssigkeitsabsorbierenden Materials in der Dickenrichtung reguliert,
ein Material des Verstärkungselements (60) ein Netz ist, in dem ein erster Faden (61) und ein zweiter Faden (62), die kreuzen, an einem Schnittpunkt dieser Fäden verbunden sind, und
das Verfahren umfassend:
Verbinden und Integrieren des Materials des luftdurchlässigen Elements (50, 50a) und des Materials des Verstärkungselements (60) durch Diffusionsschweißen des Materials des Verstärkungselements zu dem Material des luftdurchlässigen Elements unter Inertgasatmophäre an dem Schnittpunkt des ersten Fadens (61) und des zweiten Fadesn (62) während Überlagerung in der Dickenrichtung des Materials des luftdurchlässigen Elements (50, 50a) und des Materials des Verstärkungselements (60); und
Formen wenigstens eines von der Vertiefung (56a) und dem Vorsprung (56b) durch Pressen der verbundenen und integrierten Materialien des Verstärkungselements (60) und des luftdurchlässigen Elements (50, 50a) während die Materialien zwischen einer Malepressform und einer Femalepressform angeordnet sind.

8. Ein Verfahren zur Herstellung eines luftdurchlässigen Elements gemäß Anspruch 7, wobei
das Verstärkungselement (60) ein Netz ist, das durch Weben des ersten Fadens (61) und des zweiten Fadens (62) ausgebildet ist,
das luftdurchlässige Element (50) ein Netz ist, das durch Weben eines dritten Fadens (53) und eines vierten Fadens (54), die kreuzen, ausgebildet ist,
beim Verbinden und Integrieren, der erste Faden (61) und der zweite Faden (62) durch Diffusionsschweißen an dem Schnittpunkt des ersten Fadens und des zweiten Fadens verbunden sind, und
der dritte Faden (53) und der vierte Faden (54) durch Diffusionsschweißen an dem Schnittpunkt des dritten Fadens und des vierten Fadens verbunden sind.

## Revendications

1. Appareil de fabrication pour un corps absorbant (1) associé à un article absorbant, l'appareil de fabrication fabriquant le corps absorbant en utilisant un procédé dans lequel un matériau absorbant les liquides est déposé sur un élément perméable à l'air (50a) en faisant passer de l'air qui contient le matériau absorbant les liquides dans le sens de l'épaisseur de l'élément perméable à l'air (50a), l'élément perméable à l'air (50a) recouvrant une ouverture d'un élément de moule, comprenant :
un élément de renfort (60) destiné à renforcer l'élément perméable à l'air (50a) en se superposant à l'élément perméable à l'air (50a) dans le sens de l'épaisseur,
l'élément de renfort (60) étant un treillis formé en reliant un premier fil (61) et un deuxième fil (62) qui se croisent, à un point d'intersection de ces fils, et
l'élément de renfort (60) étant joint à l'élément perméable à l'air (50a) au niveau du point d'intersection, dans lequel
un corps principal de l'élément perméable à l'air (50a) est un élément plat, et
un trou de passage d'air (51a) est formé le long du sens de l'épaisseur en traversant complètement l'élément plat,
l'appareil de fabrication comprend en outre un tambour rotatif cylindrique (20) qui tourne en permanence dans un sens circonférentiel,
l'élément de moule forme une face, périphérique externe du tambour rotatif (20), la perméabilité à l'air de l'ouverture de l'élément de moule étant maintenue dans un état dans lequel il n'y aucun élément qui affecte la perméabilité à l'air à l'exception de l'élément perméable à l'air (50a) et de l'élément de renfort (60),
un espace interne et un espace externe du tambour rotatif (20) sont en communication perméable à l'air par l'intermédiaire de l'élément perméable à l'air (50a).

2. Appareil de fabrication pour un corps absorbant selon la revendication 1, dans lequel le premier fil (61) et le deuxième fil (62) sont reliés au point d'intersection par tissage.

3. Appareil de fabrication pour un corps absorbant selon la revendication 2, dans lequel le premier fil (61) et le deuxième fil (62) sont joints au point d'intersection.

4. Appareil de fabrication pour un corps absorbant selon la revendication 1, dans lequel l'élément plat comprend une région de faible densité dans laquelle la densité du trou de passage d'air est inférieure à celle d'une région environnante, à l'intérieur de l'ouverture.

5. Appareil de fabrication pour un corps absorbant selon l'une quelconque des revendications 1 à 4, dans lequel la jonction est effectuée par soudage par diffusion sous une atmosphère de gaz inerte.

6. Appareil de fabrication pour un corps absorbant selon l'une quelconque des revendications 1 à 5, lequel appareil de fabrication (10) se **caractérise en outre en ce que**
un conduit d'alimentation qui alimente en air en direction de la face périphérique externe du tambour rotatif (20) depuis l'extérieur est placé à une première position du sens circonférentiel,
une pression d'un espace interne qui est celui du tambour rotatif (20) et correspond à la première position est maintenue à une pression négative qui est inférieure à celle de l'espace externe,
lorsque l'élément de moule passe par la première position, l'air dans le conduit d'alimentation est aspiré à travers l'élément perméable à l'air (50a) dans l'espace interne du tambour rotatif (20) et le corps absorbant est déposé sur l'élément perméable à l'air (50a),
une position de retrait au niveau de laquelle le corps absorbant est retiré de l'élément perméable à l'air (50a) est établie à une position en aval de la première position dans le sens circonférentiel, et
une pression d'un espace interne qui est celui du tambour rotatif (20) et correspond à la position de retrait est maintenue à une pression supérieure ou égale à celle de l'espace externe.

7. Procédé de fabrication d'un élément perméable à l'air (50, 50a) qui est utilisé dans l'appareil de la revendication 1 lors de la formation d'un corps absorbant (1) associé à un article absorbant par dépôt d'un matériau absorbant les liquides, qui est renforcé par un élément de renfort (60), et qui forme au moins l'un parmi une dépression et une protubérance ayant une forme correspondant à une forme cible du corps absorbant (1), dans lequel
un matériau de l'élément perméable à l'air (50, 50a) est un élément régulant le passage du matériau absorbant les liquides dans le sens de l'épaisseur,
un matériau de l'élément de renfort (60) est un treillis dans lequel un premier fil (61) et un deuxième fil (62) qui se croisent sont reliés à un point d'intersection de ces fils, et
le procédé comprenant :
la jonction et l'intégration du matériau de l'élément perméable à l'air (50, 50a) et du matériau de l'élément de renfort (60) par soudage par diffusion du matériau de l'élément de renfort au matériau de l'élément perméable à l'air sous une atmosphère de gaz inerte au point d'intersection du premier fil (61) et du deuxième fil (62) en superposant dans le sens de l'épaisseur le matériau de l'élément perméable à l'air (50, 50a) et le matériau de l'élément de renfort (60) ; et
la formation d'au moins un parmi la dépression (56a) et la protubérance (56b) par pression des matériaux joints et intégrés de l'élément de renfort (60) et de l'élément perméable à l'air (50, 50a) en prenant en sandwich les matériaux entre un moule de presse mâle et un moule de presse femelle.

8. Procédé de fabrication d'un élément perméable à l'air selon la revendication 7, dans lequel
l'élément de renfort (60) est un treillis formé en tissant le premier fil (61) et le deuxième fil (62),
l'élément perméable à l'air (50) est un treillis formé en tissant un troisième fil (53) et un quatrième fil (54) qui se croisent,
par jonction et intégration, le premier fil (61) et le deuxième fil (62) sont joints par soudage par diffusion au point d'intersection du premier fil et du deuxième fil, et
le troisième fil (53) et le quatrième fil (54) sont joints par soudage par diffusion au point d'intersection du troisième fil et du quatrième fil.
